Europäisches Patentamt

European Patent Office

Office européen des brevets

⊙ Publication number: **0 274 823**
**A1**

## EUROPEAN PATENT APPLICATION

(21) Application number: 87309384.3

(22) Date of filing: 23.10.87

(51) Int. Cl.⁴ **B01D 53/34**

(30) Priority: 14.11.86 GB 8627255

(43) Date of publication of application:
20.07.88 Bulletin 88/29

(84) Designated Contracting States:
AT BE CH DE ES FR GR IT LI LU NL SE

(71) Applicant: Southern Water Authority
Guildbourne House Chatsworth Road
Worthing West Sussex BN11 1LD(GB)

(72) Inventor: Fawcett, Alan Thomas
High Firs,Wadhurst
East Sussex TN5 6EA(GB)
Inventor: Harwood, Alan
1 Church Avenue, Horsham
West Sussex RH12 2JP(GB)
Inventor: Lloyd, Anthony
Stonestile Cottage, Westfield
Hastings, East Sussex,TN35 4QR(GB)
Inventor: Slater, Phillip George
33 The Gallops, Lewes
West Sussex, BN7 1LR(GB)

(74) Representative: Kennedy, Victor Kenneth et al
G.F. REDFERN & CO. Marlborough Lodge 14
Farncombe Road
Worthing West Sussex BN11 2BT(GB)

(54) Improvements in methods and compositions for gas deodorisation.

㊼ There is described a process for deodorising foul air by contacting the air with an alkaline aqueous solution containing one or more halogens and one or more species of oxygen-containing free radical. Advantageously an aqueous alkaline solution of pH from 7 to 14 containing from 0.001% to 100% saturation of ozone, and from 0.001% to 17% available chlorine in solution is used. A liquid composition for use in the process of deodorising of foul air is also described.

EP 0 274 823 A1

# IMPROVEMENTS IN METHODS AND COMPOSITIONS FOR GAS DEODORISATION

The present invention relates to the deodorisation of gases and aerosols and is particularly concerned with the treatment of air to eliminate foul odours emitted from sewers and sewage pumping and treatment plant. The method also has utility in the deodorisation of air in other industrial plant.

The conventional treatment method to remove odour from gases. particularly sewage odours, has been to contact the foul air with a liquid so that the odour compounds are transferred to the liquid phase. The liquid may be acid or alkaline, and it is common in the art to include either ozone or chlorine in deodorisation plant scrubbing liquids to decompose the odour compounds in the liquid.

It has been suggested to use ozone in an alkaline solution as a scrubbing liquid, but this causes the ozone to decompose rapidly as was shown by M Doré in a paper presented at Imperial College. London in 1985 during the conference on "The Role of Ozone in Water and Wastewater Treatment".

It has also been shown that chlorine decreases in activity when in alkaline solution ("Disinfection and Sterilisation" by G Sykes published by Spon, page 387). This finding indicates that acidic rather than alkaline scrubbing media would be more effective vehicles for the use of chlorine in gas deodorisation.

Two disadvantages of prior techniques, utilising either chlorine or ozone as the active agent in either a neutral or alkaline vehicle. are that thiols are not completely removed from the treated air and the rate of removal of hydrogen sulphide is comparatively low.

The present invention seeks to provide a process by which substantially complete removal of not only thiol, but also others of the principal odour compounds commonly found in foul air from sewage treatment or similar plant. such as hydrogen sulphide, methane thiol dimethyldisulphide. and acetic, propionic, butyric, caproic and valeric acids may be achieved.

According to a first aspect of the present invention. a process for deodorising foul air comprises. contacting the foul air with an aqueous alkaline solution containing one or more halogens and one or more species of oxygen-containing free radicals.

Advantageously the halogen is chlorine, and the oxygen-containing free radicals are provided by adding either ozone or hydrogen peroxide directly or indirectly to the solution. By "indirectly" is meant the addition of a compound which decomposes to liberate hydrogen peroxide in the aqueous solution, such as peroxyacetic acid. Alternatively.

irradiation of the solution with ultra-violet light may be used to provide oxygen-containing free radicals.

According to a second aspect of the invention a liquid composition for use in deodorising foul air comprises an alkaline aqueous solution containing one or more halogens and one or more species of oxygen-containing free radicals.

Preferably the concentration of ozone is controlled to give a concentration of between 0.1% and 100% saturation. and the chlorine concentration is maintained to provide from 0.001% to 17% weight volume of available chlorine in alkaline solution. The pH of the solution may be from 7 to 14. and is preferably in the range of from 9 to 11.

Although the mechanism by which the process works is not totally clear, it appears that a synergistic effect arises from the presence of the halogen and the oxygen-containing free radicals which rapidly decomposes a wide range of odour compounds.

An example of the process of the invention is here exemplified by the following process steps:-

a) Ozonated air from an ozone generator is entrained in water to give a concentration of between 0.001% and 100% saturation of ozone in water. If the water is particularly hard, demineralisation may be necessary prior to ozonation.

b) In a scrubbing tower, the ozonated water is pumped from a sump at the base of the tower to the top of the tower. through which it falls over random packing back to the sump.

c) Sodium hypochlorite solution or an alkaline solution of chlorine water is added to the sump to provide available chlorine in an amount of between 0.001% and 17% weight volume in alkaline solution.

d) Foul air is fed to the base of the tower and passes upward against the counter-current of falling treated water.

As an alternative to ozonation in step a), hydrogen peroxide may be added to the water to provide oxygen-containing free radicals in solution. Similarly, in step c) bromine and iodine compounds such as hypobromides or hypoiodides may be added to the solution alone or in admixture with one or more of the chlorine providing compounds to provide the required concentration of halogen.

The ozone remaining in the treated air after leaving the tower may be monitored by an ozone analyser. and this measurement may be used to maintain a predetermined level of ozone in the treated air by controlling the ozone generator addition appropriately. Alternatively. the addition of peroxide or the operation of the ozone generator may be at a fixed rate without control of the residual peroxide or ozone in the treated air.

The efficiency of the present process will now be demonstrated by a number of comparative examples. In the following, gas chromatography (GC) analysis has been used to quantify the concentrations of odour compounds in foul air before and after treatment (see Analyst 1986 p. 1059). GC analysis has enabled the comparison of the technique against other deodorising procedures using ozone or chlorine alone dissolved in neutral or alkaline solutions.

In the following examples, GC analysis is used to identify the principal odour compounds in foul air from sewage treatment and similar installations and confirms that the present process completely removes these compounds. Thus the efficacy of the present process is more reliably assessed than can be achieved by using subjective assessment by an odour panel, particularly as ozone is known to interfere with the olfactory sense of man.

In each of the following examples, a treatment plant was used to extract 9600m³ per hour of foul air from a sewage pumping station. The foul air was drawn into a duct by means of a fan and forced through the scrubbing tower, which contained appropriate random packing, against a counter current of alkaline water containing ozone and chlorine. The flow rate of the water was either 300 litres per hour or 600 litres per hour, depending on the concentration of ozone and chlorine required for treatment of the foul air. The rate of production of ozone, when used, was controlled between 0 and 260g ozone per hour, according to a signal from an ozone analyser which was set to maintain a residual of 1ppm by volume in the treated air.

Example 1

In this example ozonated water was used without any chemical additions. The pH of the counter current water flow was 8.0. The foul air was analysed and found to contain varying levels of the odour compounds listed above. Incomplete removal of all compounds was observed in the treated air. In order to quantify the extent of removal there was introduced compressed hydrogen sulphide and methane thiol gases via a flow meter so as to give 5 vpm (parts per million by volume) of hydrogen sulphide and 0.5 vpm methane thiol in the extracted foul air flows of 9600m³ per hour. GC analysis indicated less than 70% of the hydrogen sulphide and less than 5% of the methane thiol were removed under these conditions.

Example 2

In this example the pH of the ozonated water was raised to 11 by the addition of sodium hydroxide solution and the injections of 5 vpm hydrogen sulphide and 0.5 vpm methane thiol were repeated. GC analysis indicated that 100% of hydrogen sulphide and 40% of the methane thiol was removed. Further experiments with higher concentration of hydrogen sulphide indicated that the maximum loading rate permissible for hydrogen sulphide was 7 vpm. Experiments were also carried out in which the pH was varied. It was found that a pH of at least 10 was required to ensure complete removal of hydrogen sulphide.

Example 3

In this example the ozone generator was turned off and an alkaline aqueous solution of chlorine at a flow rate of 300 litres per hour was used in the scrubbing tower. The available chlorine concentration in the water was adjusted to 330 mg per litre. Under these conditions the pH of the counter current water flow was 8.5 - 9.0. GC analysis indicated that the maximum loading rate for hydrogen sulphide was 5 vpm before the gas was detected in the outlet, and that 100% of the applied methane thiol dose of 0.5 vpm was removed.

Example 4

In this experiment the available chlorine concentration of the ozonated water was adjusted to 330 mg per litre, in accordance with the invention. The pH of the counter current water flow was 8.5 - 9.0. GC analysis indicated that the maximum loading rate of hydrogen sulphide was 10 vpm before the gas was detected in the outlet and that 100% of the applied methane thiol dose of 0.5 vpm was removed. Complete removal of other odour compounds listed in the introduction was also observed.

These examples clearly indicate that the combination of a halogen and and oxygen-containing free radical, particularly ozone and chlorine in alkaline aqueous solution, is able to achieve an improvement in odour removal as compared to either of those deodorising agents used alone. The examples also indicate that free radicals are involved in the process i.e. example 2 shows that pH in excess of 10 is required to remove hydrogen sulphide. This indicates that the removal mechanism involves dissolution of ionised hydrogen sulphide in the alkaline counter current flow. In example 4 the pH is too low for this removal process to operate and therefore a rapid free radical oxidation process must be taking place in the gas phase.

## Claims

1. A process for deodorising foul air comprising contacting the foul air with an aqueous alkaline solution containing one or more halogens and one or more species of oxygen-containing free radical.

2. A process according to claim 1, wherein the halogen is chlorine, and the oxygen-containing free radicals are provided by the addition of ozone to the aqueous solution.

3. A process according to claim 1, wherein the aqueous alkaline solution has a pH of from 7 to 14, the concentration of ozone in the solution is from 0.001% and 100% of saturation, and the concentration of chlorine is such that there is between 0.001% and 17% weight volume available chlorine in the aqueous alkaline solution.

4. A process according to claim 1 or claim 2, including the steps of:

a) Entraining ozonated air in water to give an ozone concentration of between 0.001% and 100% saturation;

b) Adding a chlorine-containing compound to the water to provide available chlorine in an amount of between 0.001% and 17% weight volume in alkaline solution and;

c) Contacting foul air with the aqueous alkaline solution of ozone and chlorine.

5. A process according to claim 1, wherein the halogen is chlorine and the oxygen-containing free radicals are provided by adding hydrogen peroxide to the aqueous solution.

6. A process according to claim 1, wherein the oxygen-containing free radicals are provided by adding hydrogen peroxide indirectly to the aqueous solution.

7. A process according to claim 6 wherein the oxygen-containing free radicals are provided by the addition of peroxyacetic acid to the aqueous solution.

8. A process according to claim 2, 3, 4, 5, 6 or 7 wherein the chlorine containing compound is sodium hypochlorite solution or an alkaline solution of chlorine water.

9. A process according to any preceding claim wherein the foul air is contacted with the aqueous alkaline solution in a counter-current scrubbing tower.

10. A liquid composition for use in deodorising foul air, comprising an aqueous alkaline solution containing one or more halogen and one ore more species of oxygen-containing free radical.

11. A liquid composition according to claim 10, wherein the halogen is chlorine and the oxygen-containing free radical is provided by the addition of ozone to the solution.

12. A liquid composition according to claim 11, wherein the pH of the solution is from 7 to 14, the concentration of ozone is from 0.001% to 100% saturation, and the concentration of available chlorine is from 0.001% to 17% weight volume.

13. A liquid composition according to claim 10, 11 or 12 wherein the pH of the solution is from 9 to 11.

14. A liquid composition according to claim 11, wherein the concentration of chlorine is 17% weight volume.

15. A liquid composition according to claim 11 wherein the concentration of ozone is greater than 0.1% saturation.

16. A process of deodorising foul air substantially as herein described.

17. A liquid composition for use in deodorising foul air substantially as herein described.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| A | EP-A-0 057 624 (CHARBONNAGES DE FRANCE) * claim 1 * | 1 | B 01 D 53/34 |
| A | CHEMISTRY AND INDUSTRY, no. 14, 18th July 1983, pages 555-558; London, GB; A.F.E. SIMS "Industrial effluent treatment with hydrogen peroxide" * page 557, right-hand column, paragraph 5 * | 1 | |
| A | PATENT ABSTRACTS OF JAPAN, vol. 5, no. 49 (C-49)[721], 8th April 1981; & JP - A - 56 5183 (MITSUBISHI DENKI) 20-01-1981 | 1 | |

TECHNICAL FIELDS SEARCHED (Int. Cl.3)

B 01 D 53/00
C 02 F 1/00

The present search report has been drawn up for all claims

| Place of search | Date of completion of the search | Examiner |
|---|---|---|
| BERLIN | 27-03-1988 | BERTRAM H E H |

EPO FORM 1503 03.82 (P0401)